Europäisches Patentamt

European Patent Office

Office européen des brevets

(11) Publication number: **0 131 252**
**B1**

# EUROPEAN PATENT SPECIFICATION

(45) Date of publication of patent specification: **06.09.89**

(51) Int. Cl.⁴: **C 07 K 7/10, A 61 K 37/02**

(21) Application number: **84107784.5**

(22) Date of filing: **04.07.84**

(54) Thymosin alpha 11.

(30) Priority: **08.07.83 US 511821**
**15.09.83 US 532418**

(43) Date of publication of application:
**16.01.85 Bulletin 85/03**

(45) Publication of the grant of the patent:
**06.09.89 Bulletin 89/36**

(84) Designated Contracting States:
**CH DE FR GB IT LI**

(56) References cited:

**PROCEEDINGS OF THE NATIONAL ACADEMY
OF SCIENCES OF THE UNITED STATES OF
AMERICA, vol. 80, no. 24, December 1983,
pages 7424-7427; J. CALDARELLA et al.:
"Thymosin alpha11: A peptide related to
thymosin alpha1 isolated calf thymosin
fraction 5"**

(73) Proprietor: **F. HOFFMANN-LA ROCHE & CO.
Aktiengesellschaft
Postfach 3255
CH-4002 Basel (CH)**

(72) Inventor: **Horecker, Bernard Leonard
340 East 64th Street Apt. 6L
New York, N.Y. (US)**

(74) Representative: **Lederer, Franz, Dr. et al
Van der Werth, Lederer & Riederer
Patentanwälte Lucile-Grahn-Strasse 22
D-8000 München 80 (DE)**

**Description**

Thymosin fraction 5, known for example from U.S. Patent 4,082,737, is a potent immunopotentiating preparation and can act to reconstitute immune functions in thymic deprived and/or immunodeprived individuals. Ongoing clinical trials with thymosin fraction 5 suggest that it is effective in increasing T cell numbers and normalizing immune function in children with thymic dependent primary immunodeficiency disease and can increase T cell numbers in immunodepressed cancer patients.

The first active peptide isolated and characterized from thymosin fraction 5 has been termed thymosin $\alpha_1$. The isolation and characterization of this peptide is described for example in U.S. Patent 4,079,127. Synthesis of thymosin $\alpha_1$ by solution and solid phase synthesis is described in U.S. Patent 4,148,788. Additionally the synthesis of thymosin $\alpha_1$ by solution phase procedures is shown in U.S. Patent 4,116,951. Thymosin $\alpha_1$ has been found to be one or more orders of magnitude more active than fraction 5 in several in vitro and in vivo assay systems designed to measure T cell differentiation and function and is currently in the clinic to determine its efficacy in the treatment of immunodeficiency diseases, of immunodepressed cancer patients and in the prevention of opportunistic infections in immunosuppressed patients.

The present invention relates to the isolation and first complete structural determination of a new polypeptide (thymosin $\alpha_{11}$) isolated from thymosin fraction 5, to pharmaceutically acceptable acid and base addition salts thereof and to pharmaceutical compositions containing this compound or its salts. Thymosin $\alpha_{11}$ has been found to have the same qualitative and quantitative biological activity as thymosin $\alpha_1$ in in vivo assay systems designed to measure T cell differentiation and function.

Thymosin $\alpha_{11}$ is a polypeptide of 35 amino acid residues, the first 28 of which are identical to thymosin $\alpha_1$. Thymosin $\alpha_{11}$ has the following amino acid sequence:

AcSer-Asp-Ala-Ala-Val-Asp-Thr-Ser-Ser-Glu-Ile-Thr-Thr-
Lys-Asp-Leu-Lys-Glu-Lys-Lys-Glu-Val-Val-Glu-Glu-Ala-Glu-
Asn-Gly-Arg-Glu-Ala-Pro-Ala-AsnOH

wherein Ac is an amino terminal acetyl group.

Thymosin $\alpha_{11}$ was isolated from calf thymus fraction 5 by a combination of preparative isoelectric focusing (see Hannappel et al., Proc. Natl. Acad. Sci. USA 79, 1708—1711 [1982]) and HPLC (see Stein and Moschera, Methods in Enzymology 79, 7—16 [1981]).

Thymosin fraction 5 prepared in accordance with known procedures, see e.g. U.S. 4,079,127, was electrofocused for 17 hours at a maximum current of 20 mA and a maximum voltage of 1.1 kV. The gel bed was divided into 30 segments (fractions) with a stainless steel grid and peptides from each segment were eluted with 5 ml of water. The pH of each eluate was determined with a pH meter.

For analysis of the peptides in the eluates by HPLC aliquots were lyophilized and dissolved in a small volume of buffer A (0.2 M pyridine, 1.0 M in formic acid). Elution was with buffer A and a linear gradient of 1-propanol.

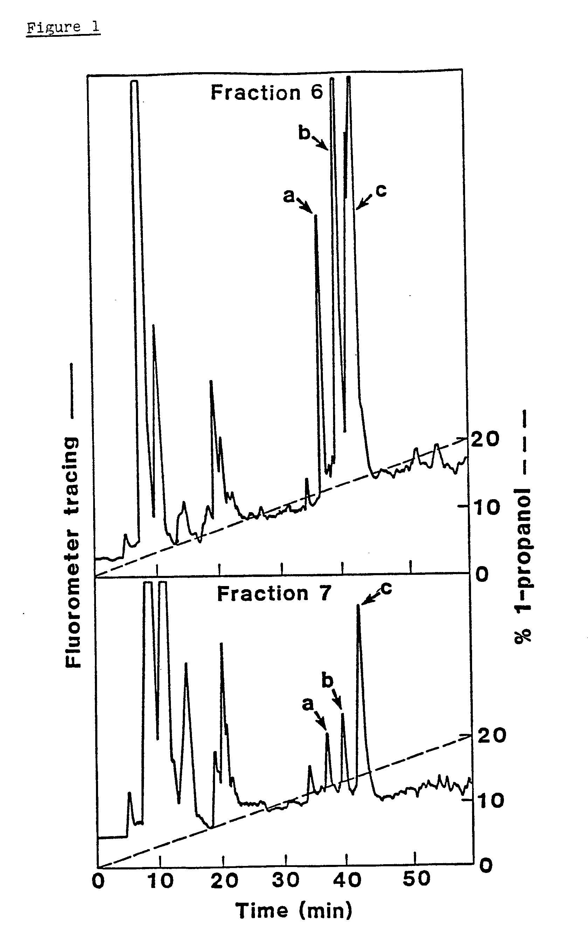

In Figure 1 results are shown for the HPLC analysis of fractions 6 and 7. Peaks a, b, and c were identified as des-(25-28)-thymosin $\alpha_1$, thymosin $\alpha_1$, and thymosin $\alpha_{11}$ respectively. Some additional thymosin $\alpha_1$ was recovered from isoelectric focusing fractions 4 and 5 and a small quantity of thymosin $\alpha_{11}$ was also present in fraction 5.

Separation of peptides by HPLC was performed with an Ultrasphere® ODS C18 column (5 µm, 4.6×250 mm, Altex Scientific) with a fluorescamine detection system as described by Stein and Moschera, supra.

The run shown in Figure 1 was derived from a 2 gram batch of calf thymosin fraction 5. The peptides in peak c of the HPLC purification step from isoelectric focusing fractions 6 and 7 were combined, lyophilized and purified by rechromatography on HPLC utilizing the methodology described for Figure 1. An aliquot (600 µg) was digested with 42.9 µg of TPCK-treated trypsin in 100 µl of 0.4 M pyridine, pH 7.5. After 15 hours at 25°C the reaction mixture was lyophilized and the tryptic peptides were separated by HPLC using a gradient of acetonitrile (0 to 30 volume percent). Fractions (0.65 ml) were collected every minute. At 6-second intervals, 5 µl samples were diverted to the fluorescamine detector.

Six fragments were recovered and identified by their amino acid composition as summarized below in Table 1.

# EP 0 131 252 B1

## TABLE 1
Amino acid composition of peptides isolated from tryptic and *S. aureus* V8
protease digests of thymosin $\alpha_{11}$

| Residue | T1 (68)* | T2 (53)* | T3 (57)* | T4 (60)* | T5 (32)* | T6 (66)* | T7 (2.6)+ | S3 (0.8)+ | S7 (3.7)+ |
|---|---|---|---|---|---|---|---|---|---|
| Asp | | 0.8 | 1.3 | 1.0 | 1.1 | 2.2 | 1.3 | 1.4 | 1.4 |
| Thr | | | | | | 2.7 | | | |
| Ser | | | | | | 2.7 | | | |
| Glu | <u>1.0</u> | <u>1.0</u> | | 3.4 | 3.3 | 1.1 | 2.4 | 4.8 | 1.7 |
| Gly | | | | 1.0 | 1.0 | | 1.4 | 1.1 | 1.9 |
| Ala | | 2.0 | | <u>1.0</u> | <u>1.0</u> | 1.9 | 2.6 | 2.7 | <u>1.0</u> |
| Val | | | | 1.9 | 1.5 | 1.1 | | 1.0 | |
| Ile | | | | | | <u>1.0</u> | | | |
| Leu | | | 0.8 | | | | | | |
| Lys | 2.1 | | <u>1.0</u> | 0.2 | | 1.0 | | 2.9 | |
| Arg | | | | 1.0 | 1.1 | | <u>1.0</u> | <u>1.0</u> | 0.5 |
| Pro | | 1.3 | | | | | nd** | nd** | nd** |

Calculated based on assigning a value of 1.0 for the residue as underlined.
* Nanomoles recovered from a digest of 200 nanomoles of thymosin $\alpha_{11}$.
+ Nanomoles recovered from a digest of 8.7 nanomoles of thymosin $\alpha_{11}$.
** Not determined.

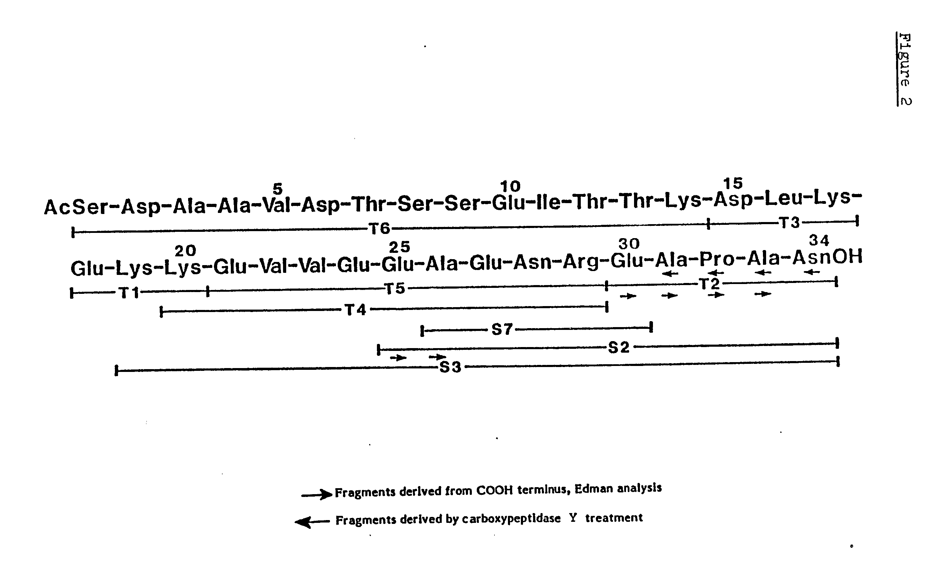

Peptides T6, T3 and T1 were identical to peptides derived from residues 1—14, 15—17 and 18—20, respectively, of thymosin $\alpha_1$. Peptides T4 and T5 were similar in amino acid composition, differing only in the presence of lysine in peptide T4. Their composition indicated that they corresponded to residues 20—28 of thymosin $\alpha_1$, plus glycine and arginine. Since peptide T5 did not contain lysine, it was concluded that arginine must be located at the COOH terminus of the peptide (see Figure 2). The tryptic digest contained an additional peptide fragment (T2) which was not present in tryptic digests of thymosin $\alpha_1$. This new peptide fragment contained no lysine or arginine, and must therefore have arisen from the COOH-terminus of thymosin $\alpha_{11}$.

Edmann degradation of tryptic peptide T2 yielded the sequence Glu-Ala-Pro-Ala-Asn-OH. This data is summarized in Table 2.

3

## TABLE 2
### Edmann degradation of tryptic peptide T2

| Step | Nanomoles of peptide recovered after each step of degradation | Subtractive method, amino acid composition of residual peptides[*] | | | | | Recovered after hydrolysis of the anilinothiazoli-none(nmol)[**] | PTH amino acid identified[+] |
|---|---|---|---|---|---|---|---|---|
| | | Glx | Ala | Pro | Ala | Asx | | |
| 0 | (35.3)[***] | 1.3 | 1.2 | 1.7 | 1.2 | 1.0 | | |
| 1 | 28.8[++] | 0.1 | 1.1 | 1.4 | 1.1 | 1.0 | Glu (6.9) | Glu |
| 2 | 30.6[++] | 0.2 | 0.1 | 1.1 | 1.2 | 1.0 | Ala (5.9) | Ala |
| 3 | 36.7[++] | 0.4 | 0 | 0.6 | 1.2 | 1.0 | Pro (2.6) | Pro |
| 4 | 30.3[++] | 0.3 | 0 | nd | 0.4 | 1.0 | Ala (2.8) | Ala |

[*] Amino acid compositions of an aliquot of thymosin $\alpha_{11}$ or of aliquots from the aqueous phase after each step of Edmann degradation. The results are presented as ratios to the quantity of aspartic acid. Half the total for alanine was arbitrarily assigned to each alanine residue in the sequence for the first 2 steps.

[**] An aliquot for each anilinothiazolinone was removed before cyclization and hydrolyzed for amino acid analysis.

[***] This quantity was used for the degradation procedure.

[+] The PTH amino acids obtained at each step of the degradation were identified by HPLC.

[++] Estimated from the results of amino acid analysis after acid hydrolysis.

Asparagine was recovered as the free amino acid after the fourth step of the Edmann procedure. Localization of peptide T2 at the COOH terminus of thymosin $\alpha_{11}$ was confirmed by digestion of the latter with carboxypeptidase (Y) which released approximately one equivalent of asparagine, followed by alanine (2 equivalents) and proline (one equivalent). The location of arginine at position 30 was confirmed by the isolation of a major fragment containing arginine after digestion of thymosin $\alpha_{11}$ with S. aureus V8 protease (peptide S7 in Table 1). The amino acid composition of this peptide corresponded to that predicted for residues 26—31 of thymosin $\alpha_{11}$ including the last four residues of thymosin $\alpha_1$, plus the first three amino acid residues, glycine, arginine and glutamic acid, found in the COOH-terminal extension of thymosin $\alpha_{11}$. Smaller quantities of two of the fragments, whose amino acid composition corresponded to residues 19—35 (peptide S3) and 25—35 (peptide S2) of thymosin $\alpha_{11}$ were also isolated from the S. aureus protease digests (Table 1 and Figure 2). The results establish thymosin $\alpha_{11}$ as containing the thymosin $\alpha_1$ sequence plus seven additional amino acids as the COOH-terminus.

The biological activity of thymosin $\alpha_1$ can be determined by utilizing in vivo assays known in the art. Thus, for example, inbred strains of mice are known to vary in their susceptibility to infection with C. albicans. Thus, mice of such strains as $C_3$ H/HeJ or CBA/CaJ are highly susceptible to infection, whereas mice of such strains as $C_{57}$ Bl/10SNJ or $C_{57}$Bl/KsJ were highly resistant to challenge. Since resistance to infection with C. albicans is associated with cell-mediated processes, and therefore with T-lymphocytes, thymic hormones should have an effect on the host response. Thymosin fraction 5 and some peptides derived therefrom have been found to enhance maturation and replication of T-lymphocytes (Goldstein et al., Rec. Progress in Hormone Research 37, 369—415 [1981]) and accordingly should influence the resistance of a susceptible murine strain, such as C₃H/HeJ, to infection with C. albicans.

Thymosin fraction 5, thymosin $\alpha_1$ or $\alpha_{11}$ was injected daily i.p. in graded doses into three different groups of mice, beginning two days before intravenous challenge with $4 \times 10^4$ cells of Candida albicans. In comparison with control mice, all three thymic derivatives provided protection. The results are summarized in Table 3 below.

4

EP 0 131 252 B1

TABLE 3

Effect of thymosin fraction 5 and thymic peptides on the growth of *Candida albicans* in C₃H/HeJ mice

| Thymosin fraction 5 | | Thymosin $\alpha_1$ | | Thymosin $\alpha_{11}$ | |
|---|---|---|---|---|---|
| Dose ng/mouse | C. albicans cell count* | Dose ng/mouse | C. albicans cell count* | Dose ng/mouse | C. albicans cell count* |
| 2560 | 8500 | 80 | 5870 | 80 | 4200 |
| 5120 | 440 | 160 | 190 | 160 | 510 |
| 10240 | 320 | 320 | 780 | 320 | 320 |
| 20480 | 1600 | 640 | 1410 | 640 | 1260 |

Mice were treated daily with the indicated doses of thymosin fraction 5, thymosin $\alpha_1$ or thymosin $\alpha_{11}$ and challenged with $4 \times 10^4$ cells of *C. albicans* two days after the start of treatment
* Three mice from each set were sacrificed on days 7, 14 and 21 after infection and the values represent the average number of organisms in the left kidneys of the nine mice in each set.

The polypeptides, thymosin $\alpha_1$ and thymosin $\alpha_{11}$, were approximately equal in potency, being most active in daily doses of 160—320 ng per mouse i.p. Since the optimum dose for fraction 5 was 5—10 µg, the peptides were about 30 times more potent than fraction 5 in their ability to induce resistance to infection with *C. albicans*.

Thus, thymosin $\alpha_{11}$, in analogy to thymosin $\alpha_1$, can be used as therapeutical agent, especially as an agent useful in the reconstitution of immune functions in thymic deprived or immunodeprived warm-blooded mammals. It may be administered in the form of pharmaceutical compositions, i.e. in mixture with a suitable pharmaceutically acceptable carrier and, if desired, further adjuvants, to warm-blooded mammals by parenteral application either intravenously, subcutaneously or intramuscularly. The compound is a potent immunopotentiating agent with a daily dosage in the range of about 1 to 100 µg/kg of body weight for intravenous administration. Obviously the required dosage will vary with the particular condition being treated, the severity of the condition and duration of the treatment. A suitable dosage form for pharmaceutical use is 1 mg of lyophilized thymosin $\alpha_{11}$ per vial to be reconstituted prior to use by the addition of sterile water or saline.

Also included within the scope of the present invention are the pharmaceutically acceptable salts of thymosin $\alpha_{11}$ such as the sodium or potassium salts or salts with strong organic bases such as guanidine. In addition, the counter ions of these cations as well as of lysine residues such as the chloride, bromide, sulphate, phosphate, maleate, acetate, citrate, benzoate, succininate, and ascorbate, may be included in the preparation.

It is easily conceivable for the skilled art worker that the sequence of thymosin $\alpha_{11}$ can be modified by single amino acid changes or that the carboxy terminus can be derivatized by ester or amide formation without essentially altering the biological activity of the thymosin $\alpha_{11}$ molecule. This is also true for desacetyl thymosin $\alpha_{11}$ which can be produced microbially, using recombinant DNA techniques, e.g. a synthetic gene which is introduced into a suitable microbial expression vehicle. After transformation with such an expression vehicle microorganisms or mammalian cells will be able to express desacetyl thymosin $\alpha_{11}$ under suitable conditions. Desacetylthymosin $\alpha_{11}$ would have the same biological activity as thymosin $\alpha_{11}$ by analogy to the relationship between thymosin $\alpha_1$ and desacetylthymosin $\alpha_1$.

**Claims**

1. Thymosin $\alpha_{11}$, a polypeptide of the sequence

AcSer-Asp-Ala-Ala-Val-Asp-Thr-Ser-Ser-Glu-Ile-Thr-Thr-
Lys-Asp-Leu-Lys-Glu-Lys-Lys-Glu-Val-Val-Glu-Glu-Ala-
Glu-Asn-Gly-Arg-Glu-Ala-Pro-Ala-AsnOH

(I)

and pharmaceutically acceptable acid and base addition salts thereof.
2. The peptide of formula I as claimed in claim 1 free of other thymic polypeptides.
3. A compound as claimed in claim 1 or claim 2 for use as therapeutical agent.
4. A compound as claimed in claim 1 or claim 2 for use as an agent reconstituting immune functions in thymic deprived or immunodeprived warm-blooded mammals.

5

5. Pharmaceutical compositions containing a compound as claimed in claim 1 or claim 2 and a suitable, pharmaceutically acceptable carrier.

6. Compositions useful in the reconstitution of immune functions in thymic deprived or immunodeprived warm-blooded mammals containing a compound as claimed in claim 1 or claim 2 and a suitable, pharmaceutically acceptable carrier.

**Patentansprüche**

1. Thymosin $\alpha_{11}$, ein Polypeptid der Sequenz

AcSer-Asp-Ala-Ala-Val-Asp-Thr-Ser-Ser-Glu-Ile-Thr-Thr-
Lys-Asp-Leu-Lys-Glu-Lys-Lys-Glu-Val-Val-Glu-Glu-Ala-
Glu-Asn-Gly-Arg-Glu-Ala-Pro-Ala-AsnOH          (I)

und pharmazeutisch annehmbare Säure- und Basen-additionssalze davon.

2. Das von anderen thymischen Polypeptiden freie, wie in Anspruch 1 beanspruchte Peptid der Formel I.

3. Eine wie in Anspruch 1 oder Anspruch 2 beanspruchte Verbindung zur Verwendung als therapeutisches Mittel.

4. Eine wie in Anspruch 1 oder Anspruch 2 beanspruchte Verbindung zur Verwendung als Mittel zur Wiederherstellung von Immunfunktionen in thymisch deprivierten oder immundeprivierten warmblütigen Säugern.

5. Pharmazeutische Zusammensetzungen die eine wie in Anspruch 1 oder Anspruch 2 beanspruchte Verbindung und einen geeigneten, pharmazeutisch annehmbaren Träger enthalten.

6. Zur Wiederherstellung von Immunfunktionen in thymisch deprivierten oder immundeprivierten warmblütigen Säugern brauchbare Zusammensetzungen, die eine wie in Anspruch 1 oder Anspruch 2 beanspruchte Verbindung und einen geeigneten, pharmazeutisch annehmbaren Träger enthalten.

**Revendications**

1. Thymosine $\alpha_{11}$, un polypeptide de séquence:

AcSer-Asp-Ala-Ala-Val-Asp-Thr-Ser-Ser-Glu-
Ile-Thr-Thr-Lys-Asp-Leu-Lys-Glu-Lys-Lys-Glu-Val-Val-Glu-Glu-
Ala-Glu-Asn-Gly-Arg-Glu-Ala-Pro-Ala-AsnOH         (I)

et ses sels d'addition d'acides et de base acceptables pharmaceutiquement.

2. Le peptide de formule I, selon la revendication 1, exempt des autres polypeptides thymiques.

3. Un composé selon la revendication 1 ou la revendication 2 pour une utilisation en tant qu'agent thérapeutique.

4. Un composé selon la revendication 1 ou la revendication 2 pour une utilisation en tant qu'agent reconstituant de fonctions immunitaires chez les mammifères à sang chaud immuno-dépressifs ou thymo-dépressifs.

5. Compositions pharmaceutiques contenant un composé selon la revendication 1 ou la revendication 2 et un véhicule acceptable pharmaceutiquement.

6. Compositions utiles pour la reconstitution des fonctions immunitaires chez les mammifères à sang chaud immuno-dépressifs ou thymo-dépressifs contenant un composé selon la revendication 1 ou la revendication 2, et un véhicule approprié acceptable pharmaceutiquement.

Figure 1

Figure 2

AcSer-Asp-Ala-Ala-Val-Asp-Thr-Ser-Ser-Glu-Ile-Thr-Thr-Lys-Asp-Leu-Lys-
Glu-Lys-Lys-Glu-Val-Val-Glu-Glu-Ala-Glu-Asn-Arg-Glu-Ala-Pro-Ala-AsnOH

Fragments derived from COOH terminus, Edman analysis

Fragments derived by carboxypeptidase Y treatment